# EUROPEAN PATENT SPECIFICATION

(11) **EP 0 791 041 B1**
(45) Date of publication and mention of the grant of the patent: **11.04.2001**
(21) Application number: 95925223.0
(22) Date of filing: 03.07.1995
(51) Int. Cl.: C11C 3/12

(54) **HYDROGENATION OF SUBSTRATE WITH SUBSTRATE AND HYDROGEN IN A SUBSTANTIALLY HOMOGENEOUS SUPER-CRITICAL OR NEAR-CRITICAL SOLUTION**
HYDRIERUNG EINES SUBSTRATES MIT SUBSTRAT UND WASSERSTOFF IN EINER IM WESENTLICHEN HOMOGENEN, ÜBERKRITISCHEN ODER ANNÄHERND KRITISCHEN LÖSUNG
PROCEDE D'HYDROGENATION DU SUBSTRAT, LE SUBSTRAT ET L'HYDROGENE ETANT DANS UNE SOLUTION SUBSTANTIELLEMENT HOMOGENE SUPERCRITIQUE OU PRESQUE CRITIQUE

(30) Priority: 01.07.1994 SE 9402362; 28.03.1995 SE 9501159
(43) Date of publication of application: 27.08.1997
(73) Proprietor: POUL MÖLLER LEDELSES- OG INGENIÖRRRADGIVNING APS, 8000 Arhus (DK); Härröd, Magnus, 441 65 Alingsas (SE)
(72) Inventor: HÄRRÖD, Magnus, 441 65 Alingsas (SE); MÖLLER, Poul, 8000 Arhus (DK)
(74) Representative: Roth, Ernst Adolf Michael
(86) International application number: SE9500824
(87) International publication number: WO9601304

(56) References cited:
- WO-A-94/06738
- WO-A-95/22591
- DE-A- 4 405 029
- US-A- 3 969 382
- Proceedings of the Third International Symposium on Supercritical Fluids

## Description

### TECHNICAL FIELD OF THE INVENTION.

The present invention relates to a process for the hydrogenation of a substrate, where hydrogen gas is mixed with the substrate in the presence of a catalyst and the reaction is carried out at certain reaction conditions of pressure, time and temperature. The hydrogenation reactions are mainly related to the hydrogenation of carbon-carbon double bonds (C=C) in lipids;
hydrogenation of COOR to C-OH and HO-R for the manufacturing of fatty alcohols; and the direct hydrogenation of oxygen to hydrogen peroxide.

### BACKGROUND OF INVENTION.

### C=C in lipids.

The annual production of vegetable oils is about 90 million tons (Mielke 1992), of which about 20% are hardened (hydrogenated). Furthermore, about 2 million tons of marine oils are hydrogenated yearly. The production is spread over the whole industrialized world. Through the hydrogenation, hydrogen is added to the double bonds of the unsaturated fatty acids. The largest part of the oils is only partly hydrogenated. The desired conditions of melting and the desired consistency of the fats are thereby obtained, which are of importance for the production of margarine and shortening. The tendency to oxidation is reduced by the hydrogenation, and the stability of the fats is increased at the same time (Swern 1982).

In the future, the lipids may be modified by methods belonging to bio technology, especially gene technology, but hydrogenation will certainly remain.

A problem with the hydrogenation processes of today is, that new fatty acids are produced which to a great extent do not exist in the nature. They are often called trans fatty acids, but the double bonds change position as well as form (cis-trans) during the hydrogenation (Allen 1956, Allen 1986).

As a rule, trans fatty acids are desired from a technical and functional point of view (Swern 1982), but regarding health, their role is becoming more and more questionable (Wahle & James 1993).

A typical state of the art reactor for hydrogenation is a large tank (5 to 20 m³) filled with oil and hydrogen gas plus a catalyst in the form of fine particles (nickel in powdery form). The reaction is carried out at a low pressure, just above atmospheric (0,5 to 5 bar), and high temperatures (130 to 210°C). The hydrogen gas is thoroughly mixed into the oil, as this step restricts the velocity of the reaction rate (Grau et al., 1988).

If the pressure of hydrogen gas is increased from 3 to 50 bar when soya oil is partially hydrogenated (iodine number at the start = 135, at the end = 70), the content of trans is reduced from 40 to 15 %. The position isomerization is also reduced to a corresponding level (Hsu et al., 1989). These results are of no commercial interest, as these conditions enforce a replacement of the low pressure autoclaves by high pressure autoclaves.

According to the "half hydrogenation" theory, the con-ccntration of activated H-atoms on the catalyst surface determines the number of double bonds being hydrogenated and deactivated without being hydrogenared respectively. A lack of activated H-atoms causes a trans and position- isomerization (Allen 1956, Allen 1986). A lack of activated H-atoms can be the consequence of low solubility of H2 in the oil, or of a bad catalyst (poisoned or inadequately produced). Thus, the "half hydrogenation" theory corresponds very well to the empirical results (Allen 1956; Allen 1986; Hsu et al., 1989).

It is possible to deodorize and hydrogenate an oil in the presence of CO2 and hydrogen (Zosel 1976). Zosel describes in detail how to use CO2 in order to deodorize the oil. However, it must be emphasized that Zosel does not give any hint, that CO2 should have an influence on the hydrogenation process. Furthermore, Zosel does not touch on the cis/trans problem.

In the experiments of Zosel, the catalyst is surrounded by an liquid phase during the entire process. Zosel does not disclose the composition, but in the light of the other data, we estimate that the liquid phase consists of oil (about 95%), CO2 (about 5%) and hydrogen (about 0,03%). This phase is far away from a super critical condition. As a consequence, the velocity of reaction is limited by the concentration of hydrogen on the catalyst surface. The same applies to all traditional hydrogenation reactions where the catalyst is in the liquid phase as well. The velocity of hydrogenation in the experiments of Zosel is about 100 kg/m3h, i.e. somewhat lower than in traditional hydrogenizing reactors.

### FATTY ALCOHOLS.

Fatty alcohols and their derivatives are used in shampoo, detergent compositions and cosmetic preparations etc. The annual production is about 1 million tons. About 60% is based on petrochemicals, and about 40% is derived from natural fats and oils. The raw material for short chain fatty alcohols, C12-C14, is coco-nut oil and palm kern oil, whereas C16-C18 comes from tallow, palm oil or palm stearin (Kreutzer 1984, Ong et al., 1989).

It is theoretically possible to hydrogenate triglycerides, fatty acids and methyl esters to fatty alcohols. A direct hydrogenation of triglycerides has not been developed commercially, because the glycerol will be hydrogenated as well and thus lost. A direct hydrogenation of fatty acids requires corrosion resistant materials and a catalyst resistant to acids (Kreutzer 1984). Lurgi has developed a hydrogenation process (the slurry process), where fatty acids are introduced and are quickly esterified with a fatty alcohol to a wax ester, and then the wax ester is hydrogenated (copper chromite, 285°C, 300 bar)(Buchhold 1983, Voeste Buchhold 1984, Lurgi 1994).

Most plants for the production of natural fatty alcohols are based on methyl esters as raw material. Saturated fatty alcohols are produced at a temperature of about 210°C and a pressure of 300 bar using copper chromite as catalyst in a fixed bed reactor. Other catalysts as copper carbonate, nickel or copper and chromic oxide will also function (Mahadevan 1978, Monick 1979, Lurgi 1994). Unsaturated fatty alcohols are produced at about 300°C and 300 bar, normally using zinc chromite as catalyst. There are also other catalysts which selectively hydrogenate the group COOR, leaving the C=C unimpaired (Klonowski et al., 1970; Kreutzer 1984).

The reaction is limited by the solubility of hydrogen in the liquid (Hoffman Ruthhardt 1993).

Davy Process Technology markets a gas phase process where methyl esters are hydrogenated to fatty alcohols (40 bar, 200 to 250°C, catalyst without chromium) (Hi].es 1994).

A lot of work has been done to develop catalysts functioning with less energy (lower temperature, lower pressure). Another object has been to develop methods for a direct hydrogenation of triglycerides to fatty alcohols without a simultaneous hydrogenation of the glycerol (Hoffman Ruthhardt 1993).

### HYDROGEN PEROXIDE.

Hydrogen peroxide is used in large quantities for bleaching, cleaning, as a disinfectant and as a raw material in industrial processes etc. Earlier, hydrogen peroxide was derived by an electrolytic process. Now, oxidation of substituted hydroquinone or 2-propanol is most widely used.

There are a lot of patents concerned with direct synthesis of hydrogen peroxide from oxygen and hydrogen. The reaction medium can be acidic organic solvents or water with organic solvents using noble metals, most often palladium, as catalyst (EP-B-0049806; EP-B-0117306; US-A-4336239; EP-B-0049809).

It is preferred that the reaction medium is free from organic constituents because of problems with purification. Several patents use acidic water as the reaction medium (pH = 1-2) with addition of halides, especially bromide and chloride (<1 mM) and with noble metals or mixtures of noble metals as catalysts (EP-A-0132294; EP-A-0274830; US-A-4393038; DE-B-2655920; DE 4127918 Al).

The velocities of reaction which are disclosed are about 1 kg/m3h, and the selectivity (mol hydrogen peroxide/mol hydrogen reacted) is about 75% (DE 4127918 Al).

According to theory, one can expect to obtain high selectivity with high concentrations of oxygen and hydrogen on the catalyst surface (Olivera et al., 1994).

### THE OBJECT OF THE INVENTION AND MOST IMPORTANT CHARACTERISTICS.

The object of the present invention is to obtain a very effective process for partial or complete hydrogenation of the substrates mentioned above.

According to the invention, this problem has been solved by mixing the substrate, hydrogen gas and solvent, and by bringing the whole mixture in to a super-critical or near-critical state to form a substantially homogeneous super-critical or near-critical solution. This substantially homogeneous super-critical or near-critical solution is led over the catalyst, whereby the reaction products formed, i.e. the hydrogenated substrates, will also be a part of the substantially homogeneous super-critical or near-critical solution.

Having regard to the designated state Germany, the invention relates to a process for the hydrogenation of a substrate, excluding hydrogenation of fats, fatty acids or fatty acid esters, in which only the carbon-carbon-double bonds (C=C) are hydrogenated, where hydrogen gas is mixed with the substrate in the presence of a catalyst, and the reaction is carried out at selected conditions of pressure, time and temperature,
**characterized in** that substrate, hydrogen gas and a solvent are mixed together, the mixture is brought to a super-critical or near-critical state to form a substantially homogeneous super-critical or near-critical solution, and the substantially homogeneous super-critical or near-critical solution is brought into contact with the catalyst, and also in that the reaction products, i.e the hydrogenated substrates, form a constituent in the super-critical or near-critical solution.

The solvent can be a saturated hydrocarbon or an unsaturated hydrocarbon which on hydrogenation gives a saturated hydrocarbon, e.g. ethane, ethene, propane, propene, butane, butene, or CO2, dimethyl ether, "freons", N2O, N2, NH3, or mixtures thereof.

Propane is a suitable solvent for many lipids. CO2 is a suitable solvent for hydrogen peroxide and water.

The catalyst will be selected according to the reaction which is to be carried out. For a partial or complete hydrogenation of only C=C bonds, preferably a noble metal or nickel will be selected. For a selective hydrogenation of COOR to C-OH and HO-R, the catalyst would preferably be a zinc salt, e.g. zinc chromite. For a simultaneous hydrogenation of COOR to C-OH and HO-R and a hydrogenation of C=C, the preferred catalyst would be copper chromite, another salt of copper or copper free from chrome. For a partial hydrogenation of oxygen to hydrogen peroxide, the preferred catalyst would be a noble metal.

According to the invention, the concentration of hydrogen on the catalyst surface can be controlled to very high levels. The proportion of trans fatty acids in partially hydrogenated fatty products will be much lower according to the invention than by using conventional processes, where the product has been hydrogenated to the same level using the same catalyst. The hydrogenated products will preferably contain less than 10% trans fatty acids.

### DESCRIPTION OF THE DRAWINGS.

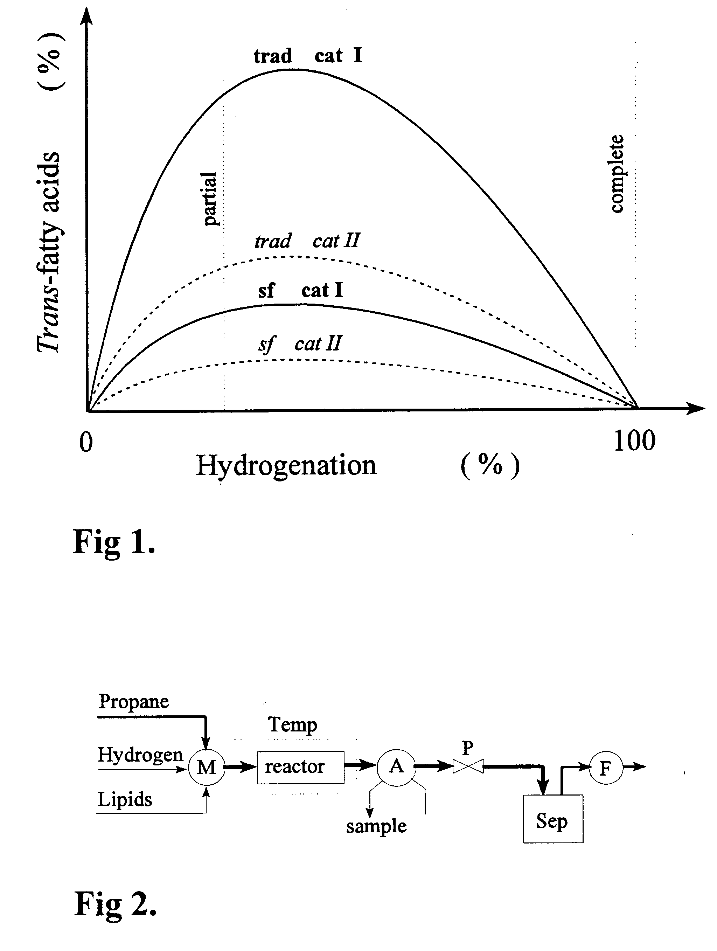

Figure 1 is a diagram showing the percentage of trans fatty acids as a function of the degree of hydrogenation according to a traditional technique and according to the invention. Figure 2 is a flow sheet for a process according to the invention.

### DESCRIPTION OF THE INVENTION.

The problem. In a great number of hydrogenation processes, hydrogen gas is mixed with a liquid substrate and a fixed catalyst, e.g. in the hydrogenation of lipids. In certain cases the substrate can be a gas and the product a liquid, e.g. hydrogenation of oxygen to hydrogen peroxide and water. In both these cases, the velocity of reaction is limited by the concentration of gas on the catalyst surface. The reason is the transport resistances of the gas: between the gas phase and the liquid phase; through the liquid phase; and between the liquid phase and the catalyst.

### THE SUBJECT MATTER OF THE INVENTION.

The idea is to add a solvent, which completely dissolves the gas as well as the liquid, resulting in a substantially homogeneous mixture of hydrogen, substrate, product and solvent. This is possible, if the whole mixture is in a super-critical or near-critical state. The defition substantially homogeneous means, that the principal part of the gas is in the continuous phase which covers the catalyst surface. One method to confirm this is to observe the velocity of reaction, which increases dramatically when the continuous phase that covers the catalyst surface is substantially homogeneous.

### VELOCITY OF REACTION.

According to the invention, the following transport resistances of the gas are reduced substantially: gas phase/liquid phase; through the liquid phase; and liquid phase/catalyst. The velocity of reaction thereby increases to a very high degree; from about 10 to about 1000 times. The consequence of this is that continuous reactors will be preferred compared to the batch reactors of to-day. The selectivity is also influenced to a very high degree.

### SOLVENT.

In order to bring the whole mixture (hydrogen, substrate, product and solvent) to super-critical or near-critical state at appropriate pressures and temperatures, the solvent must dissolve substrate and product as much as possible.

Glycerides, fatty acids and many derivatives of fatty acids are completely miscible with super-critical propane (Peter et al.,1993). Propane can be used in any proportions together with food according to EU-regulations (Sanders 1993; EC 1984). Thus, propane is a very adequate solvent in reactions with lipids.

Water dissolves to a certain extent in CO2 (King et al., 1992). Hydrogen peroxide dissolves more easily than water in CO2. Thus, CO2 is an appropriate solvent for direct synthesis of hydrogen peroxide. (For a thorough description of super-critical technology, see McHugh Krakonis 1986; Dohrn 1994).

### CATALYSTS.

The catalysts which are used today in traditional
processes can in principle also be used in super-critical processes. The catalyst may however be modified to optimize selectivity, velocity of reaction, length of life, filtering properties and pressure-drop.

### QUALITY OF PRODUCT.

The invention, enables new possibilities to control the hydrogen concentration at the catalyst. The velocity of reaction increases substantially. The selectivity can also be influenced in certain processes. By partial hydrogenation of edible oils, the content of trans fatty acids is of importance for the quality (see background of invention).

Figure 1 illustrates in principle how the proportion of trans fatty acids changes during hydrogenation with two different catalysts, one catalyst according to a traditional technique and another according to the new super-critical technique. The new super-critical technique makes it possible to reduce the content of trans fatty acids in comparison with the traditional technique using the same catalyst and the same degree of hydrogenation. However, using different catalysts, the difference may be less.

In Figure 1, "trad" means traditional process; and "sf" means process with super critical fluid. "cat" means catalyst.

### CONDITIONS OF REACTION.

### C=C in lipids.

I. Partial hydrogenation. At partial hydrogenation, the reaction is interrupted at a certain iodine number, e.g. 60. The substrate, e.g. vegetable, animal or marine oil, and hydrogene are dissolved in a solvent, e.g. propane. The mixture is brought to a super-critical or a near-critical state. The substantially homogeneous mixture is brought into contact with a catalyst, e.g. palladium. The content of trans fatty acids in the final product is less than 10%. The optimal reaktion condition may occure over a wide experimental range and this range can be described as follows:

| | | |
|---|---|---|
| -temperature | 0 - 250°C | 20 - 200°C |
| -pressure | 10 - 350 bar | 20 - 200 bar |
| -time of reaction | 0^{*} - 10 min | 1 µsec- 1 min |
| -solvent | 30 - 99, 9wt% | 40 - 99wt% |

The solvent must dissolve the substrates at the concentrations used. The solvent can be ethane, ethene, propane, propene, butane, butene, CO2, dimethyl ether, "freons", N2O, N2, NH3 or mixtures of these gases. Preferred are propane, propene, butane, butene and dimethyl ether. Most preferred is propane.
- concentration of H2 0^{*} - 3wt% 0,001 - lwt%
- concentr. substrate 0,1 - 70wt% 1 - 60wt%
- type of substrate C=C in general; glycerides are preferred
   (mono-, di-, triglycerides, galactolipids, phospholipids), also fatty acids or their derivatives (e.g. methyl- and ethyl-esters).
- catalysts noble metals: Pd, Pt, Os, but also Ni. (O^{*} means very low values, below the lowest one under "preferably").

### II. COMPLETE HYDROGENATION.

At complete hydrogenation, all double bonds are hydrogenated and the iodine number is therefore near zero. The substrate, e.g. vegetable, animal or marine oil, and hydrogen are dissolved in a solvent, e.g. propane. The mixture is brought to a supercritical or near-critical condition, and the substantially homogeneous mixture is brought into contact with a catalyst, e.g. palladium.
The optimal conditions of reaction are wide and can be described in a similar way as for partial hydrogenation; the temperature is, however, somewhat higher than for partial hydrogenation (T is probably higher than Tcrit).

### FATTY ALCOHOLS.

The substrate, e.g. the triglyceride, the fatty acid or its derivative, and hydrogen are mixed together with a solvent, e.g. propane. The mixture is brought to a super-critical or a near-critical state, and the substantially homogeneous mixture is brought into contact with a catalyst. Different groups can be hydrogenated depending on the catalyst used (see below under "-catalyst").

The optimal reaction condition may occure over a wide experimental range and this range can be described as follows:

| | | |
|---|---|---|
| | in general | preferably |
| -temperature | 20 - 300°C | 40 - 300°C |
| -pressure | 10 - 350 bar | 20 - 200 bar |
| -time of reaction | 0^{*} - 10 min | 1µsec- 1 min |
| -solvent | 30 - 99,9wt% | 40 - 99wt% |

The solvent must dissolve the substrates at the concentrations used. The solvent can be ethane, ethene, propane, propene, butane, butene, CO2, dimethyl ether, "freons", N2O, N2, NH3 or mixtures of these gases. Preferred are propane, propene, butane, butene, and dimethyl ether. Sometimes, it can be advantageous to use an entrainer. Most preferred is pure propane.
- concentration H2 0^{*} - 3wt% 0,001 - lwt%
- concentr. substrate 0,1 - 70wt% 1 - 60 wt%
- type of substrate: COOR in general. Preferred are fatty acids and their derivatives (e.g. methyl-ethyl- or wax esters), and also mono- di-, and tri-glycerides, but also galac- tolipids and phospholipids.
- catalyst a)selective hydrogenation of COOR, but not C=C or C-OH, e.g. zinc chromite or
another salt of zinc.

b ) hydrogenation of both COOR and C=C but not C-OH, e.g. copper chromite or copper free from chrome or an other salt of copper.
(0^{*} means very low values, less than the lowest under "preferably").

An example of suitable values at optimal conditions is: substrate 10 wt%, propane about 90 wt%, hydrogen 0,2 wt%; the mixture is brought into contact with a bed of catalyst at 250°C and 150 bar, and has an average contact time of 30 sec.

### HYDROGEN PEROXIDE.

Oxygen and hydrogen are mixed in a solvent, e.g. CO2. The mixture is brought to a super-critical or near-critical state, and the substantially homogeneous mixture is brought in contact with a catalyst. The solvent dissolves the reaction products, hydrogen peroxide and water. Thus, a substantially homogeneous mixture is maintained in the reactor.

The optimal reaction condition may occure over a wide experimental range and this range can be described as follows:

| | | |
|---|---|---|
| | in general | preferably |
| -temperature | 10 - 200°C | 20 - 10 0 ° C |
| -pressure | 10 - 350 bar | 30 - 300 bar |
| -time of reaction | 0^{*} - 10 min | 1µsec- 1 min |
| -solvent | 10 - 99, 9wt% | 60 - 99wt% |

The solvent must dissolve water and hydrogen peroxide at the concentrations used. The solvent can be CO2, N2, NH3, or mixtures of these gases. It may also be advantageous to use an entrainer. Pure CO2 is probably the most suitable solvent.

| | | |
|---|---|---|
| -concentration H2 | 0* - 10wt% | 0,1 - 3wt% |
| -concentration O2 | 0,1 - 80wt% | 1 - 30wt% |
| -catalyst | | noble metals, e.g. Pd or mixtures of noble metals, |
| e.g. Pd + Au | | |
| -reaction aids | | halides, e.g. bromides or chlorides; these can be |

added in the process or at the preparation of the catalyst (O^{*} means very low values, less than the lowest under "preferably")

The risk of explosion during some of the processing steps must be warned against.

Suitable proportions of the added constituents can be exemplified by: oxygen 3wt%, hydrogen O,lwt% and CO2 96,9wt%. The mixture is brought into contact with a catalyst of palladium at 35°C and 200 bar; the average contact time is 0,1 sec.

### EQUIPMENT AND ANALYTICAL METHODS

### Equipment

A flow sheet for the continuous reactor used, is illustrated in Figure 2. In this figure "M" is a mixer, "Temp." a temperature controller, "A" a sampling device for analyses, "P" a pressure reduction valve, "Sep" a vessel for separation of gas/liquids and "F" a gasflow-meter. At room temperature a condensed gas, a non-condensable gas and a liquid were mixed according to the principles used by Pickel in a "Supercritical Fluid Chromatography" application (Pickel 1991). Pickel mixed CO₂ nitrogen and a liquid entrainer. We mixed propane (1), hydrogen (g) and lipids (see M in Figure 2). The same equipment can be used for the hydrogen peroxide experiments but in this case one add:
CO₂ (1); oxygen + hydrogen (g); recation acides (l).

The mixture was heated to the desired reaction temperature and was brought into an HPLC tube filled with a catalyst powder (see Temp and Reactor in Figure 2).

After the reactor samples were collected from the high pressure section using an HPLC valve (see A in Figure 2 and Härröd et al 1994).

The pressure was reduced to atmospheric pressure and lipids and gases were separated (see P and Sep in Figure 2). The gasflow was controlled by the pressure-reduction valve (P om Figure 2).

### Analysis

The product quality was analysed using silver-ion-HPLC and gradient elution (Elfman Härröd 1995). This method is developed from an isocratic method (Adolf 1994). The kind (cis/trans) and the amount of the fatty acid methyl esters (FAME) was determine. From these data the iodine value (IV) was calculated.

The density was calculated from the Peng-Robinsson equation of state (Dohrn 1994).

### EXAMPLES

### Example 1

### Partial hydrogenation of methylesters from rapeseed oil using a palladium catalyst.

| Composition and amound of the inlet flow to the reactor: | | | |
|---|---|---|---|
| | mole% | weight% | mg/min |
| propane | 99.92 | 99.7 | 3700 |
| hydrogen | 0.04 | 0.002 | 0.07 |
| FAME | 0.04 | 0.26 | 10 |

| Reactio conditions: | |
|---|---|
| catalyst | 5% Pd on char coal (E 101 O/D 5% Degussa AG) |
| reactor volume | 0.0007 ml |
| reaction time | 40 ms |
| temperature | 50 °C |
| pressure | 120 bar |

productivity and product quality:
productivity 80 000 kg FAME/m³ h
Iodine-value reactor inlet=110 reactor outlet=50
FAME with trans 10% of all FAME

### Comments

This example shows that a very high productivity (80 000 kg FAME/m³ h) and a low content of trans-fatty acids (10%) can be attained at near-critical conditions. The results above is only an example. We do not claim that it is the optimal conditions for the process.

Others (Berben et al 1995) has minimized the trans-fatty acid content using the conventional technique. The productivity became much lower (700 kg triglycerides /m³ h) and the content of the trans-fatty acids became much higher (34%).

Example 2

### Complete hydrogenation of methylesters from rapseed oil using a palladium catalyst.

| Composition and amount of the inlet flow to the reactor: | | | |
|---|---|---|---|
| | mole% | weight% | mg/min |
| propane | 96.27 | 95.7 | 1840 |
| hydrogen | 3.1 | 0.14 | 2.7 |
| FAME | 0.63 | 4.16 | 80 |

| Reaction conditions: | |
|---|---|
| catalyst | 5% Pd on char coal (E 101 O/D 5% Degussa AG) |
| reactor volume | 0.007 |
| reaction time | 80 ms |
| temperature | 90 °C |
| pressure | 70 bar |

productivity and product quality:
productivity. 700 000 kg FAME/m³ h
Iodine-value reactor inlet=110 reactor outlet <1
FAME with trans <0.1% of all FAME

### Comments

This example shows that a tremendous productivity (700 000 kg FAME/m³ h) can be attained at near-critical conditions. The results above is only an example. We do not claim that it is the optimal conditions for the process.

### Example 3

### Complete hydrogenation of methylesters from rapeseed oil using a nickel catalyst.

| Composition and amount of the inlet flow to the reactor: | | | |
|---|---|---|---|
| | mole% | weight% | mg/min |
| propane | 99.49 | 99.13 | 1500 |
| hydrogen | 0.38 | 0.017 | 0.25 |
| FAME | 0.13 | 0.85 | 13 |

| Reaction conditions: | |
|---|---|
| catalyst | Nickel (Ni-5256 P, Engelhard) |
| reactor volume | 0.009 ml |
| reaction time | 65 ms |
| temperature | 190 °C |
| pressure | 155 bar |

productivity and product quality:
productivity 90 000 kg FAME/m³ h
Iodine-value reactor inlet=110 reactor outled <1
FAME with trans <0.1% of all FAME

### Comments

This example shows that a very high productivity (90 000 kg FAME/m³ h) can be attained using a nickel catalystat super-critical conditions. The results above is only an example. We do not claim that it is the optimal conditions for the process.

Example 4

### Complete hydrogenation of triglycerides using a palladium catalyst.

| Composition and amount of the inlet flow to the reactor: | | | |
|---|---|---|---|
| | mole% | weight% | mg/min |
| propane | 98.7 | 93.6 | 3600 |
| hydrogen | 1 | 0.043 | 1.6 |
| triglycerides | 0.03 | 6.3 | 240 |

The triglycerides (tg) were in this case a commercial vegetable oil.

| Reaction conditions: | |
|---|---|
| catalyst | 5% Pd on char coal (E 101 O/D 5% Degussa AG) |
| | |
| reactor volume | 2.5 ml |
| reactor time | 12 sec |
| temperature | 50 °C |
| pressure | 100 bar |

productivity and product quality:
productivity 5000 kg tg/m³ h
Iodine-value reactor inlet=140 reactor outlet =0.1
FA with trans <0.1% of all FA

### Comments:

This example shows that a high productivity (5000 kg triglycerides/m³ h) can be attained at near-critical conditions. The results above is only an example. We do not claim that it is the optimal conditions for the process.

### REFERENCES

Adlof R.O. 1994 *J.Chromatography* A 659, p95-99.
Allen R.R., Kiess A.A., 1956, JACOS, 33(aug), 355-359
Allen R.R., 1986, JAOCS, 63(10), 1328-1332
Berben P.H., P.J.W. Blom and J.C. Sollie 1995. In *Proceedings of AOCS meeting, May 1995*
Buchhold H, 1983, Chem. Eng., Feb., 42-43
Dohrn R. 1994 *Berechnungen von Phasengleichgewichten*, Vieweg, Braunschweig /Wiesbaden, Germany
EC, 1984, EC directive 84 /344 / EEC
Elfman 1. and M. Härröd 1995. (submitted)
Grau R.J., Cassano A.E., Baltanas M.A., 1988, Catal. Rev. -Sci. Eng., 30(1), 1-48
Hiles A., 1994, Oils & Fats International, 10(2), 15-18
Hoffman M., Ruthardt K., 1993, Oils &Fats International, 9(2), 26-30.
Hsu N., Diosady L.L., Rubin L.J., 1989, JACOS, 66(2), 232-236
Härröd M., M. Lilja-Hallberg and I. Elfman 1994. In *Proceedings of 3*^{*rd*} *international symposium on supercritical fluids*. (eds Brunner G.H. and M. Perrut) INPL, Nancy, France, Part 3, p. 149.
King M.B., A. Mubarak, J.D. Kim, T.R. Bott, 1992, *J. Supercritical Fluids*, 5(4), 296-302.
Klonowski R.S., Findley T.W., Josefson C.M., Stirton A.J., 1970, *JAOCS*, 47(Sept), 326-328.
Kreutzer U.R., 1984, JAOCS, 61(2), 343-348
Lurgi, 1994, Fatty Alcohol Technology, Lurgi, Frankfurt am Main, Tyskland, 235e /3.94/20
Mahadevan V., 1978, *In* Progress in the Chemistry of Fats and other Lipids. (Holman R.T. ed ) Pergamon Press, Oxford, UK. 15(4). 255-299.
McHugh M.A. V. Krukonis.1986, *Supercritical fluid extraction*, Butterworths, Boston, Mass..US A
Mielke S., 1992, INFORM. 3(6), 695-696
Monick J.A., 1979, JAOCS, 56, 853A-861A
Olivera P. P., Patrito E.M. och Sellers H., 1994. Surface Science, 313, 25-40
Ong A.S.H., Cheah K. Y., Choo Y.M., 1989, Elaeis 1(1),31-51
Peter S., Weidner E., Czech B., Ender U.. 1993, Fat. Sci, Technol., 95, 475-482
Pickel K.H. 1991 In *Proceedings of 2*^{*nd*} *international symposium on supercritical fluids*. (ed
McHugh M.) Johns Hopkins University. Baltimore, MD, p.457.

Sanders N., 1993, In *Extraction of Natural Products using near-critical solvents* (King M.B., Bott T.R. eds.), Chapman & Hall, London, p.35-49
Swern D., 1982, Bailey's industrial oil and fat products, Vol.2, 4th ed., Wileys, New York
Voeste T., Buchold H., 1984, JAOCS, 61(2), 350-352.
Wahle K.W.J., James W.P.T., 1993, European J. Clinical Nutrition, 47, 828-839
Wisniak J.. Albright L.F., 1961, Ind. Eng. Chem., 53(5), 375-380
Zosel K., 1976, patent US, A, 3969382,

## Claims (Claims for the following Contracting State(s): AT, BE, CH, DK, ES, FR, GB, IT, LI, NL, PT, SE)

1. A process for the hydrogenation of a substrate, where hydrogen gas is mixed with the substrate in the presence of a catalyst, and the reaction is carried out at selected conditions of pressure, time and temperature,
**characterized in**
that substrate, hydrogen gas and a solvent are mixed together, the mixture is brought to a super-critical or near-critical state to form a substantially homogeneous super-critical or near-critical solution, and the substantially homogeneous super-critical or near-critical solution is brought into contact with the catalyst, and also in that the reaction products, i.e the hydrogenated substrates, form a constituent in the super-critical or near-critical solution.

2. A process according to claim 1,
**characterized in**
that the solvent comprises saturated hydrocarbon or an unsaturated hydrocarbon, which results on hydrogenation in a saturated hydrocarbon, e.g. ethane, ethene, propane, propene, butane, butene, or CO2, dimethyl ether, "freons", N₂O, N₂, NH₃, or mixtures thereof.

3. A process according to claim 1 or 2,
**characterized in**
that the substrate comprises lipids such as triglycerides, fatty acids or derivatives of fatty acids.

4. A process according to claim 3,
**characterized in**
that the solvent is propane, propene, butane, butene or dimethyl ether or mixtures thereof.

5. A process according to claim 2 or 4,
**characterized in**
that the solvent preferably is propane.

6. A process according to any of the preceding claims,
**characterized in**
that preferably a nobel metal or a nickel catalyst is used for the hydrogenation of only the carbon-carbon-double bonds (C=C).

7. A process according to any of the preceding claims,
**characterized in**
that the reaction is interruped when the desired iodine number has been obtained, which means a iodine number near zero for full hydrogenation and above zero for partial hydrogenation.

8. A process according to any of claims 1-5,
**characterized in**
that preferably zinc chromite or any salt of zinc is used as a catalyst for the hydrogenation of COOR to C-OH and/or HO-R while the carbon-carbon double bonds (C=C) are not hydrogenated.

9. A process according to any of claims 1-5,
**characterized in**
that preferably copper chromite or copper free from chrome, or any other salt of copper is used as a catalyst for the hydrogenation of COOR to C-OH and HO-R and the hydrogenation of C=C.

10. A process according to claim 1 or 2,
**characterized in**
that the substrate comprises oxygen.

11. A process according to claim 10,
**characterized in**
that the solvent comprises CO₂, N₂, NH₃, or mixtures thereof.

12. A process according to claim 11,
**characterized in**
that the solvent preferably is CO₂.

13. A process according to any of claims 10-12,
**characterized in**
that preferably a noble metal is used as a catalyst for the hydrogenation of oxygen to hydrogen peroxide.

## Claims (Claims for the following Contracting State(s): DE)

1. A process for the hydrogenation of a substrate, excluding hydrogenation of fats, fatty acids or fatty acid esters, in which only the carbon-carbon-double bonds (C=C) are hydrogenated, where hydrogen gas is mixed with the substrate in the presence of a catalyst, and the reaction is carried out at selected conditions of pressure, time and temperature,
**characterized in**
that substrate, hydrogen gas and a solvent are mixed together, the mixture is brought to a super-critical or near-critical state to form a substantially homogeneous super-critical or near-critical solution, and the substantially homogeneous super-critical or near-critical solution is brought into contact with the catalyst, and also in that the reaction products, i.e the hydrogenated substrates, form a constituent in the super-critical or near-critical solution.

2. A process according to claim 1,
**characterized in**
that the solvent comprises saturated hydrocarbon or an unsaturated hydrocarbon, which results on hydrogenation in a saturated hydrocarbon, e.g. ethane, ethene, propane, propene, butane, butene, or CO₂, dimethyl ether, "freons", N₂O, N₂, NH₃, or mixtures thereof.

3. A process according to claim 1 or 2,
**characterized in**
that the substrate comprises lipids such as triglycerides, fatty acids or derivatives of fatty acids, wherein COOR is hydrogenated to C-OH and/or HO-R, but-the carbon-carbon double bonds (C=C) are not hydrogenated.

4. A process according to claim 3,
**characterized in**
that preferably zinc chromite or any salt of zinc is used as a catalyst for the hydrogenation of COOR to C-OH and/or HO-R but the carbon-carbon double bonds (C=C) are not hydrogenated.

5. A process according to claim 1 or 2,
**characterized in**
that the substrate comprises lipids such as triglycerides, fatty acids or derivatives of fatty acids, wherein COOR is hydrogenated to C-OH and/or HO-R as well as the carbon-carbon double bonds (C=C) are hydrogenated.

6. A process according to claim 5,
**characterized in**
that preferably copper chromite or copper free from chrome, or any other salt of copper is used as a catalyst for the hydrogenation of COOR to C-OH and HO-R and the hydrogenation of C=C.

7. A process according to any of claims 3-6,
**characterized in** that the solvent is propane, propene, butane, butene or dimethyl ether or mixtures thereof.

8. A process for the hydrogenation of only the carbon-carbon-double bonds (C=C) in a substrate in the form of lipids, fatty acids or fatty acid derivatives, where hydrogen gas is mixed with the substrate in the presence of a catalyst,
**characterized, in**
that at least 0.1% by weight, preferably at least 1% by weight, substrate, at most 3% by weight, preferably at most 1% by weight hydrogen gas and at least 30% by weight, preferably at least 40% by weight of a solvent, are mixed together, the mixture is brought to a super-critical or near-critical state at a temperature between 0 and 250°C and a pressure between 10 and 350 bar, to form a substantially homogeneous super-critical or near-critical solution, and the substantially homogeneous super-critical or near-critical solution is brought into contact with the catalyst at a period of time of at most 10 minutes, preferably at most 1 minute, and also in that the reaction products, i.e. the hydrogenated substrates, form a constituent in the super-critical or near-critical solution.

9. A process according to claim 8,
**characterized in**
that the solvent is propane, propene, butane, butene or dimetyl ether.

10. A process according to claim 9,
**characterized in**
that the solvent preferably is propane.

11. A process according to any of claims 1 or 8-10,
**characterized in**
that preferably a nobel metal or a nickel catalyst is used for the hydrogenation of only cabon-carbon-double bonds (C=C).

12. A process according to any of claims 1 or 8-11,
**characterized in**
that the reaction is interruped when the desired iodine number has been obtained, which means a iodine number near zero for full hydrogenation and above zero for partial hydrogenation.

13. A process according to claim 1 or 2,
**characterized in**
that the substrate comprises oxygen.

14. A process according to claiml3,
**characterized in**
that the solvent comprises CO₂, N₂, NH₃, or mixtures thereof.

15. A process according to claim 14,
**characterized in**
that the solvent preferably is CO₂.

16. A process according to any of claims 13-15,
**characterized in**
that preferably a noble metal is used as a catalyst for the hydrogenation of oxygen to hydrogen peroxide.

## Patentansprüche (Patentansprüche für folgende(n) Vertragsstaat(en): AT, BE, CH, DK, ES, FR, GB, IT, LI, NL, PT, SE)

1. Verfahren für die Hydrierung eines Substrats, wobei Wasserstoffgas mit dem Substrat in Gegenwart Katalysators gemischt wird und die Reaktion bei ausgewählten Druck-, Zeit- und Temperaturbedingungen durchgeführt wird,
**dadurch gekennzeichnet,**
daß Substrat, Wasserstoffgas und ein Lösungsmittel zusammengemischt werden und die Mischung in einen superkritischen oder nahe-kritischen Zustand gebracht wird, um eine im wesentlichen homogene superkritische oder nahe-kritische Lösung zu bilden und die im wesentlichen homogene superkritische oder nahe-kritische Lösung mit dem Katalysator in Kontakt gebracht wird und auch dadurch, daß die Reaktionsprodukte, d.h. die hydrierten Substrate, einen Bestandteil in der superkritischen oder nahe-kritischen Lösung bilden.

2. Verfahren nach Anspruch 1,
**dadurch gekennzeichnet,**
daß das Lösungsmittel gesättigten Kohlenwasserstoff oder einen ungesättigten Kohlenwasserstoff aufweist, der beim Hydrieren zu einem gesättigten Kohlenwasserstoff führt, z.B. Ethan, Ethen, Propan, Propen, Butan, Buten, oder CO₂, Dimethylether, "Freone", N₂O, N₂, NH₃, oder Mischungen davon.

3. Verfahren nach Anspruch 1 oder 2,
**dadurch gekennzeichnet,**
daß das Substrat Lipide, wie Triglyceride, Fettsäuren oder Derivate von Fettsäuren aufweist.

4. Verfahren nach Anspruch 3,
**dadurch gekennzeichnet,**
daß das Lösungsmittel Propan, Propen, Butan, Buten oder Dimethylether oder Mischungen davon ist.

5. Verfahren nach Anspruch 2 oder 4,
**dadurch gekennzeichnet,**
daß das Lösungsmittel vorzugsweise Propan ist.

6. Verfahren nach einem der vorhergehenden Ansprüche,
**dadurch gekennzeichnet**,
daß vorzugsweise ein Edelmetall- oder Nickelkatalysator zur Hydrierung von nur den Kohlenstoff-Kohlenstoff-Doppelbindungen (C=C) verwendet wird.

7. Verfahren nach einem der vorhergehenden Ansprüche,
**dadurch gekennzeichnet**,
daß die Reaktion unterbrochen wird, wenn die erwünschte lodzahl erreicht worden ist, d.h. eine lodzahl nahe Null für vollständige Hydrierung und über Null für Teilhydrierung.

8. Verfahren nach einem der Ansprüche 1 bis 5,
**dadurch gekennzeichnet,**
daß vorzugsweise Zinkchromit oder jedes Zinksalz als Katalysator für die Hydrierung von COOR zu C-OH und/oder HO-R verwendet wird, während die Kohlenstoff-Kohlenstoff-Doppelbindungen (C=C) nicht hydriert werden.

9. Verfahren nach einem der Ansprüche 1 bis 5,
**dadurch gekennzeichnet**,
daß vorzugsweise Kupferchromit oder chromfreies Kupfer oder jedes andere Kupfersalz als Katalysator für die Hydrierung von COOR zu C-OH und HO-R und die Hydrierung von C=C verwendet wird.

10. Verfahren nach Anspruch 1 oder 2,
**dadurch h gekennzeichnet,**
daß das Substrat Sauerstoff aufweist.

11. Verfahren nach Anspruch 10,
**dadurch gekennzeichnet,**
daß das Lösungsmittel CO₂, N₂, NH₃ oder Mischungen davon aufweist.

12. Verfahren nach Anspruch 11,
**dadurch h gekennzeichnet,**
daß das Lösungsmittel vorzugsweise CO₂ ist.

13. Verfahren nach einem der Ansprüche 10 bis 12,
**dadurch gekennzeichnet,**
daß vorzugsweise Edelmetall als Katalysator für die Hydrierung von Sauerstoff zu Wasserstoffperoxid verwendet wird.

## Patentansprüche (Patentansprüche für folgende(n) Vertragsstaat(en): DE)

1. Verfahren für die Hydrierung eines Substrates, ausschließlich Hydrierung von Fetten, Fettsäuren oder Fettsäureestern, wobei nur die Kohlenstoff-Kohlenstoff-Doppelbindungen (C=C) hydriert werden, wobei Wasserstoffgas mit dem Substrat in Gegenwart eines Katalysators gemischt wird und die Reaktion bei ausgewählten Druck-, Zeit- und Temperaturbedingungen durchgeführt wird,
**dadurch gekennzeichnet,**
daß Substrat, Wasserstoffgas und ein Lösungsmittel zusammen gemischt werden und die Mischung in einen superkritischen oder nahe-kritischen Zustand gebracht wird, um eine im wesentlichen homogene superkritische oder nahe-kritische Lösung zu bilden und die im wesentlichen homogene superkritische oder nahe-kritische Lösung mit dem Katalysator in Kontakt gebracht wird und auch dadurch, daß die Reaktionsprodukte, d.h. die hydrierten Substrate, einen Bestandteil in der superkritischen oder nahe-kritischen Lösung bilden.

2. Verfahren nach Anspruch 1,
**dadurch gekennzeichnet,**
daß das Lösungsmittel gesättigten Kohlenwasserstoff oder einen ungesättigten Kohlenwasserstoff aufweist, der beim Hydrieren zu einem gesättigten Kohlenwasserstoff führt, z.B. Ethan, Ethen, Propan, Propen, Butan, Buten, oder CO₂, Dimethylether, "Freone", N₂O, N₂, NH₃, oder Mischungen davon.

3. Verfahren nach Anspruch 1 oder 2,
**dadurch gekennzeichnet,**
daß das Substrat Lipide, wie Triglyceride, Fettsäuren oder Derivate von Fettsäuren aufweist, worin COOR zu C-OH und/oder HO-R hydriert wird, jedoch die Kohlenstoff-Kohlenstoff-Doppelbindungen (C=C) nicht hydriert werden.

4. Verfahren nach Anspruch 3,
**dadurch gekennzeichnet,**
daß vorzugsweise Zinkchromit oder jedes Zinksalz als Katalysator für die Hydrierung von COOR zu C-OH und/oder HO-R verwendet wird, während die Kohlenstoff-Kohlenstoff-Doppelbindungen (C=C) nicht hydriert werden.

5. Verfahren nach Anspruch 1 oder 2,
**dadurch gekennzeichnet,**
daß das Substrat Lipide, wie Triglyceride, Fettsäuren oder Derivate von Fettsäuren aufweist, worin COOR zu C-OH und/oder HO-R hydriert wird, und auch die Kohlenstoff-Kohlenstoff-Doppelbindungen (C=C) hydriert werden.

6. Verfahren nach Anspruch 5,
**dadurch gekennzeichnet**,
daß vorzugsweise Kupferchromit oder chromfreies Kupfer oder jedes andere Kupfersalz als Katalysator für die Hydrierung von COOR zu C-OH und/oder HO-R und die Hydrierung von C=C verwendet wird.

7. Verfahren nach einem der Ansprüche 3-6,
**dadurch gekennzeichnet,**
daß das Lösungsmittel Propan, Propen, Butan, Buten oder Dimethylether oder Mischungen davon ist.

8. Verfahren für die Hydrierung von nur den Kohlenstoff-Kohlenstoff-Doppelbindungen (C=C) in einem Substrat in Form von Lipiden, Fettsäuren oder Fettsäurederivaten, wobei Wasserstoffgas mit dem Substrat in Gegenwart eines Katalysators gemischt wird,
**dadurch gekennzeichnet,**
daß mindestens 0,1 Gewichts-% Substrat, höchstens 3 Gewichts-%, bevorzugt höchstens 1 Gewichts-% Wasserstoffgas und mindestens 30 Gewichts-%, bevorzugt mindestens 40 Gewichts-% eines Lösungsmittels zusammengemischt werden, die Mischung in einen superkritischen oder nahe-kritischen Zustand bei einer Temperatur zwischen 0 und 250°C und einem Druck zwischen 10 und 350 bar gebracht wird, um eine im wesentlichen homogene superkritische oder nahe-kritische Lösung zu bilden und die im wesentlichen homogene superkritische oder nahe-kritische Lösung in Kontakt mit dem Katalysator während einer Zeitdauer von höchstens 10 Minuten, vorzugsweise höchstens 1 Minute gebracht wird und auch dadurch, daß die Reaktionsprodukte, d.h. hydrierte Substrate, einen Bestandteil in der superkritischen oder nahe-kritischen Lösung bilden.

9. Verfahren nach Anspruch 8,
dadurch gekennzeichnet,
daß das Lösungsmittel Propan, Propen, Butan, Buten oder Dimethylether ist.

10. Verfahren nach Anspruch 9,
**dadurch gekennzeichnet,**
daß das Lösungsmittel vorzugsweise Propan ist.

11. Verfahren nach einem der Ansprüche 1 oder 8 bis 10,
**dadurch gekennzeichnet,**
daß vorzugsweise ein Edelmetall oder ein Nickelkatalysator für die Hydrierung von nur den Kohlenstoff-Kohlenstoff-Doppelbindungen (C=C) verwendet wird.

12. Verfahren nach einem der Ansprüche 1 oder 8 bis 11,
**dadurch gekennzeichnet,**
daß die Reaktion unterbrochen wird, wenn die erwünschte lodzahl erreicht worden ist, d.h. eine lodzahl nahe Null für vollständige Hydrierung und über Null für Teilhydrierung.

13. Verfahren nach Anspruch 1 oder 2,
**dadurch gekennzeichnet**,
daß das Substrat Sauerstoff aufweist.

14. Verfahren nach Anspruch 13,
**dadurch gekennzeichnet,**
daß das Lösungsmittel CO₂, N₂, NH₃ oder Mischungen davon aufweist.

15. Verfahren nach Anspruch 14,
**dadurch gekennzeichnet,**
daß das Lösungsmittel vorzugsweise CO₂ ist.

16. Verfahren nach einem der Ansprüche 13-15,
**dadurch gekennzeichnet,**
daß vorzugsweise ein Edelmetall als Katalysator für die Hydrierung von Sauerstoff zu Wasserstoffperoxid verwendet wird.

## Revendications (Revendications pour l'(les) Etat(s) contractant(s) suivant(s): AT, BE, CH, DK, ES, FR, GB, IT, LI, NL, PT, SE)

1. Procédé d'hydrogénation d'un substrat, où de l'hydrogène gazeux est mélangé au substrat en présence d'un catalyseur, et la réaction est effectuée à des conditions sélectionnées de pression, de temps et de température,
caractérisé en ce que
le substrat, l'hydrogène gazeux et un solvant sont mélangés ensemble, le mélange est amené dans un état supercritique ou presque critique pour former une solution supercritique ou presque critique pratiquement homogène, et la solution supercritique ou presque critique pratiquement homogène est mise en contact avec le catalyseur, et également en ce que les produits de réaction, c'est-à-dire les substrats hydrogénés, forment un composé de la solution supercritique ou presque critique.

2. Procédé selon la revendication 1, caractérisé en ce que le solvant comporte un hydrocarbure saturé ou un hydrocarbure insaturé, qui a pour résultat une hydrogénation en hydrocarbure saturé, par exemple de l'éthane, de l'éthène, du propane, du propène, du butane, du butène, ou CO₂, de l'éther diméthylique, des "fréons", N₂O, N₂, NH₃, ou des mélanges de ceux-ci.

3. Procédé selon la revendication 1 ou 2, caractérisé en ce que le substrat comporte des lipides tels que des triglycérides, des acides gras ou des dérivés d'acide gras.

4. Procédé selon la revendication 3, caractérisé en ce que le solvant est du propane, du propène, du butane, du butène, ou de l'éther diméthylique ou des mélanges de ceux-ci.

5. Procédé selon la revendication 2 ou 4, caractérisé en ce que le solvant est de préférence du propane.

6. Procédé selon l'une quelconque des revendications précédentes, caractérisé en ce que, de préférence, un métal noble ou un catalyseur de nickel est utilisé pour l'hydrogénation uniquement des doubles liaisons carbone-carbone (C=C).

7. Procédé selon l'une quelconque des revendications précédentes, caractérisé en ce que la réaction est interrompue lorsque l'indice d'iode voulu a été obtenu, ce qui signifie un indice d'iode proche de zéro pour une hydrogénation complète et au-dessus de zéro pour une hydrogénation partielle.

8. Procédé selon l'une quelconque des revendications 1 à 5, caractérisé en ce que, de préférence, de la chromite de zinc ou tout sel de zinc est utilisée en tant que catalyseur pour l'hydrogénation de COOR en C-OH et/ou HO-R alors que les doubles liaisons carbone-carbone (C=C) ne sont pas hydrogénées.

9. Procédé selon l'une quelconque des revendications 1 à 5, caractérisé en ce que, de préférence, de la chromite de cuivre ou du cuivre exempt de chrome, ou tout autre sel de cuivre, est utilisée en tant que catalyseur pour l'hydrogénation de COOR en C-OH et HO-R et l'hydrogénation de C=C.

10. Procédé selon la revendication 1 ou 2, caractérisé en ce que le substrat comporte de l'oxygène.

11. Procédé selon la revendication 10, caractérisé en ce que le solvant comporte CO₂, N₂, NH₃, ou des mélanges de ceux-ci.

12. Procédé selon la revendication 11, caractérisé en ce que le solvant est de préférence CO₂.

13. Procédé selon l'une quelconque des revendications 10 à 12, caractérisé en ce que, de préférence, un métal noble est utilisé en tant que catalyseur pour l'hydrogénation d'oxygène en peroxyde d'hydrogène.

## Revendications (Revendications pour l'(les) Etat(s) contractant(s) suivant(s): DE)

1. Procédé d'hydrogénation d'un substrat, à l'exception de graisses, d'acides gras ou d'esters d'acides gras, dans lequel seules les doubles liaisons carbone-carbone (C=C) sont hydrogénées, où de l'hydrogène gazeux est mélangé au substrat en présence d'un catalyseur, et la réaction est effectuée à des conditions sélectionnées de pression, de temps et de température,
caractérisé en ce que
le substrat, l'hydrogène gazeux et un solvant sont mélangés ensemble, le mélange est amené dans un état supercritique ou presque critique pour former une solution supercritique ou presque critique pratiquement homogène, et la solution supercritique ou presque critique pratiquement homogène est mise en contact avec le catalyseur, et également en ce que les produits de réaction, c'est-à-dire les substrats hydrogénés, forment un composé de la solution supercritique ou presque critique.

2. Procédé selon la revendication 1, caractérisé en ce que le solvant comporte un hydrocarbure saturé ou un hydrocarbure insaturé, qui a pour résultat une hydrogénation en hydrocarbure saturé, par exemple de l'éthane, de l'éthène, du propane, du propène, du butane, du butène, ou CO₂, de l'éther diméthylique, des "fréons", N₂O, N₂, NH₃, ou des mélanges de ceux-ci.

3. Procédé selon la revendication 1 ou 2, caractérisé en ce que le substrat comporte des lipides tels que des triglycérides, des acides gras ou des dérivés d'acide gras, dans lequel COOR est hydrogéné en C-OH et/ou HO-R, mais les doubles liaisons carbone-carbone (C=C) ne sont pas hydrogénées.

4. Procédé selon la revendication 3, caractérisé en ce que, de préférence, de la chromite de zinc ou tout sel de zinc est utilisée en tant que catalyseur pour l'hydrogénation de COOR en C-OH et/ou HO-R mais les doubles liaisons carbone-carbone (C=C) ne sont pas hydrogénées.

5. Procédé selon la revendication 1 ou 2, caractérisé en ce que le substrat comporte des lipides tels que des triglycérides, des acides gras ou des dérivés d'acides gras, dans lequel COOR est hydrogéné en C-OH et/ou HO-R, et les doubles liaisons carbone-carbone (C=C) sont également hydrogénées.

6. Procédé selon la revendication 5, caractérisé en ce que, de préférence, de la chromite de cuivre ou du cuivre exempt de chrome, ou tout autre sel de cuivre, est utilisée en tant que catalyseur pour l'hydrogénation de COOR en C-OH et HO-R et l'hydrogénation de C=C.

7. Procédé selon l'une quelconque des revendications 3 à 6, caractérisé en ce que le solvant est du propane, du propène, du butane, du butène ou de l'éther diméthylique ou des mélanges de ceux-ci.

8. Procédé d'hydrogénation uniquement des doubles liaisons carbone-carbone (C=C) dans un substrat ayant la forme de lipides, d'acides gras ou de dérivés d'acides gras, où de l'hydrogène est mélangé au substrat en présence d'un catalyseur,
caractérisé en ce que
au moins 0,1 % en poids de substrat, au plus 3 % en poids, de préférence au plus 1 % en poids, d'hydrogène gazeux et au moins 30 % en poids, de préférence au moins 40 % en poids, d'un solvant, sont mélangés ensemble, le mélange est amené dans un état supercritique ou presque critique à une température comprise entre 0 et 250°C et à une pression comprise entre 10 et 350 bars, pour former une solution supercritique ou presque critique pratiquement homogène, et la solution supercritique ou presque critique pratiquement homogène est mise en contact avec le catalyseur sur une période de temps d'au plus 10 minutes, de préférence d'au plus 1 minute, et également en ce que les produits de réaction, c'est-à-dire les substrats hydrogénés, forment un composé de la solution supercritique ou presque critique.

9. Procédé selon la revendication 8, caractérisé en ce que le solvant est du propane, du propène, du butane, du butène ou de l'éther diméthylique.

10. Procédé selon la revendication 9, caractérisé en ce que le solvant est de préférence du propane.

11. Procédé selon l'une quelconque des revendications 1 ou 8 à 10, caractérisé en ce que, de préférence, un métal noble ou un catalyseur de nickel est utilisé pour l'hydrogénation uniquement des doubles liaisons carbone-carbone (C=C).

12. Procédé selon l'une quelconque des revendications 1 ou 8 à 11, caractérisé en ce que la réaction est interrompue lorsque l'indice d'iode voulu a été obtenu, ce qui signifie un indice d'iode proche de zéro pour une hydrogénation complète et au-dessus de zéro pour une hydrogénation partielle.

13. Procédé selon la revendication 1 ou 2, caractérisé en ce que le substrat comporte de l'oxygène.

14. Procédé selon la revendication 13, caractérisé en ce que le solvant comporte CO₂, N₂, NH₃, ou des mélanges de ceux-ci.

15. Procédé selon la revendication 14, caractérisé en ce que le solvant est de préférence CO₂.

16. Procédé selon l'une quelconque des revendications 13 à 15, caractérisé en ce que, de préférence, un métal noble est utilisé en tant que catalyseur pour l'hydrogénation d'oxygène en peroxyde d'hydrogène.
